# FASCICULE DE BREVET EUROPEEN

(11) **EP 1 053 742 B1**
(45) Date de publication et mention de la délivrance du brevet: **04.08.2004**
(21) Numéro de dépôt: 00401244.9
(22) Date de dépôt: 05.05.2000
(51) Int. Cl.: A61K 7/02, A61K 7/027, A61K 7/48, A61K 7/035

(54) **Compositions anhydres de soin ou de maquillage contenants des fibres et des polyols**
Wasserfreie Make-up oder Hautpflegemittel die Fasern und Polyole enthalten
Anhydrous skin care or make-up compositions containing fibres and polyols

(30) Priorité: 20.05.1999 FR 9906411
(43) Date de publication de la demande: 22.11.2000
(73) Titulaire: L'OREAL, 75008 Paris (FR)
(72) Inventeur: Jager-Lezer, Nathalie, 92340 Bourg-la-Reine (FR)
(74) Mandataire: Lhoste, Catherine

(56) Documents cités:
- EP-A- 0 534 823
- EP-A- 0 838 210
- WO-A-98/19652
- FR-A- 2 675 995
- CHEMICAL ABSTRACTS, vol. 115, no. 18, 4 novembre 1991 (1991-11-04) Columbus, Ohio, US; abstract no. 189495, ONDA, EIZO: "Liquid mascara composition containing dyed fibers" XP002131513 & JP 03 153613 A (ISEHAN K. K., JAPAN) 1 juillet 1991 (1991-07-01)
- DATABASE WPI Section Ch, Week 198245 Derwent Publications Ltd., London, GB; Class A96, AN 1982-96016E XP002131467 & JP 57 158714 A (SHISEIDO CO LTD), 30 septembre 1982 (1982-09-30)
- DATABASE WPI Section Ch, Week 199539 Derwent Publications Ltd., London, GB; Class D21, AN 1995-299461 XP002131468 & JP 07 196440 A (SHISEIDO CO LTD), 1 août 1995 (1995-08-01)

## Description

La présente invention se rapporte à une composition anhydre contenant des fibres, destinée aux domaines cosmétique et dermatologique. Plus spécialement, l'invention s'applique au soin et/ou au traitement et/ou maquillage des matières kératiniques comme la peau, y compris le cuir chevelu, les lèvres et les phanères comme les cils, les sourcils, les ongles et les cheveux des êtres humains.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eyes liners, les mascaras, les compositions de soin, de protection solaire ou de coloration de la peau, de maquillage du corps ou encore de maquillage des cheveux.

Il est connu d'utiliser des fibres dans des produits de maquillage notamment pour leurs effets allongeant dans des mascaras (voir JP-A-57/158714), leur toucher " textile" (voir JP-A-7/196440), leur effet de tissus ou encore leurs propriétés hydratantes dans des rouges à lèvres (voir le document US-A-5 498 407) ou pour améliorer les contours du rouge à lèvres sur les bords des lèvres (voir le document EP-A-0 106 762). Malheureusement, il est très difficile de disperser des fibres dans des compositions, de façon homogène et sans former d'amas, ce qui dans une composition colorée et en particulier de maquillage confère généralement un maquillage non uniforme et peu esthétique. En outre, cette difficulté de dispersion conduit à des compositions de propriétés cosmétiques non constantes et peu reproductibles, ce qui entraîne des problèmes de fabrication industrielle et des coûts élevés de fabrication.

Les produits de maquillage ou de soin de la peau ou des lèvres des êtres humains comme les fonds de teint ou les rouges à lèvres contiennent généralement des phases grasses telles que des cires et des huiles, des pigments et/ou charges et éventuellement des additifs comme des actifs cosmétiques ou dermatologiques.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

De plus, ces compositions ont tendance à migrer, c'est-à-dire à se propager à l'intérieur des rides et des ridules de la peau qui entourent les lèvres et les yeux, sur lesquels sont appliquées ces compositions, entraînant un effet inesthétique. Par migration, on entend un débordement de la composition, en particulier de la couleur, hors du tracé initial.

Dans la demande JP-A-61-65809 la société Shiseido a décrit des compositions de rouge à lèvres "sans transfert" contenant une résine siloxysilicate (à réseau tridimensionnel), une huile de silicone volatile à chaîne silicone cyclique et des charges pulvérulentes. Par ailleurs, la société Noevier a décrit dans le document JP-A-62-61911 des compositions de rouge à lèvres, d'eye liner et de fonds de teint "sans transfert" comportant une ou plusieurs silicones volatiles associées à une ou plusieurs cires hydrocarbonées.

Ces compositions, bien que présentant des propriétés de "sans transfert" améliorées ont l'inconvénient de laisser sur les lèvres, après évaporation des huiles de silicone, un film qui devient inconfortable au cours du temps (sensation de dessèchement et de tiraillement), écartant un certain nombre de femmes de ce type de rouge à lèvres.

En outre, ces compositions à base d'huiles de silicones volatiles et de résines siliconées conduisent à des films colorés mats. Or, la femme est aujourd'hui à la recherche de produits notamment de coloration des lèvres, brillants. De plus, les propriétés de "sans transfert" des films déposés ne sont pas parfaites. En particulier, une pression ou un frottement prononcé, conduit à une diminution de la couleur du dépôt et à un redépôt sur le support mis en contact avec ces films.

Il subsiste donc le besoin d'une composition ne présentant pas les inconvénients ci-dessus, et ayant notamment de bonnes propriétés de tenue, de "sans transfert", même lors d'une pression ou d'un frottement prononcé, un aspect plus ou moins brillant, adapté au désir de la consommatrice, ne migrant pas, ne desséchant pas la peau ou les lèvres sur lesquelles elle est appliquée, aussi bien lors de l'application qu'au cours du temps et conférant un maquillage ou un soin homogène esthétique.

L'invention a justement pour objet une composition de soin et/ou de traitement et/ou de maquillage des matières kératiniques permettant de remédier à ces inconvénients. De façon surprenante, le demandeur a trouvé que l'utilisation de fibres dans une composition de maquillage conférait à la composition de bonnes propriétés de tenue, de sans transfert et de non-migration, tout en étant confortable à porter et d'aspect satiné à brillant et que l'utilisation de polyols permettait de disperser de façon homogène ces fibres dans la composition, et donc de conférer un maquillage homogène et harmonieux.

L'invention s'applique non seulement aux produits de maquillage de la peau, aussi bien du visage que du corps humain, et des lèvres mais aussi aux produits de maquillage des phanères comme les cils, sourcils, ongles et cheveux ainsi qu'aux produits de soin et/ou de traitement de la peau, y compris du cuir chevelu.

De façon plus précise, l'invention a pour objet une composition anhydre de soin ou de maquillage des matières kératiniques, contenant des fibres compatibilisées avec une phase grasse par au moins un polyol liquide à température ambiante les fibres ayant une longueur allant de 10 nm à 5 mm, et une section comprise dans un cerle de diamètre allant de 2 nm à 100 µm.

Par "composition anhydre", il faut comprendre une composition comprenant une phase grasse continue homogène, dans laquelle peuvent être dispersés des ingrédients insolubles dans ladite phase grasse, en l'absence de tensio-actif ou émulsionnant, comme des matières colorantes, des actifs cosmétiques ou dermatologiques, y compris de l'eau. En particulier, l'eau sera présente à une teneur au plus égale à 6 % du poids total de la composition, et par exemple inférieure à 2 % et mieux inférieur à 0,5 %.

Par "phase grasse", il faut comprendre un milieu non aqueux, non miscible à l'eau, contenant un ou plusieurs corps gras choisis parmi les composés ayant au moins 10 atomes de carbone et mieux 16 atomes de carbone, les composés siliconés, les composés fluorés et leurs mélanges. Ne sont pas considérés comme corps gras les solvants organiques utilisés classiquement dans les vernis à ongles.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500 et mieux de 5 à 150.

Par "température ambiante", il faut comprendre une température de 25°C, à pression atmosphérique normale (76 mm de Hg).

Par "polyol", il faut comprendre toute molécule organique comportant au moins deux groupements hydroxyle libres. En particulier, le ou les polyols de l'invention présentent une valeur d'IOB (Balance/Inorganique/Organique) supérieure à 0,5 et en particulier allant de 1 à 7 et plus spécialement de 1,5 à 5,5.

Le paramètre IOB est connu de l'homme du métier à partir d'un certain nombre de publications comme l'article de A. FUJITA Pharm. Bull 2, 163-173 (1954) et les documents JO 9/151109, J08/217639 de Shiseido ou J09/175925 de Kose.

A titre d'exemples de polyols qui vérifient les critères précédents, et qui peuvent être utilisés seuls ou en mélange dans la composition de l'invention, on peut citer :

| **Nom** | **Valeur d'IOB** |
|---|---|
| - Propylène glycol | 3,333 |
| - Butylène glycol | 2,500 |
| - Isoprène glycol | 2,222 |
| - Pentylène glycol | 2,000 |
| - Hexylène glycol | 1,818 |
| - PEG-4 (*) | 2,656 |
| - PEG-6 | 2,396 |
| - PEG-8 | 2,266 |
| - Glycérol | 5,000 |
| - Panthénol | 3,125 |

| | |
|---|---|
| (*) De façon générale, on peut citer les polyéthylène glycols (PEG) ayant de 4 à 8 motifs d'éthylène glycol. | |

A titre d'exemple de polyols qui ne vérifient pas le critère précédent relatif à l'IOB, on peut citer:

| **Nom** | **Valeur d'IOB** |
|---|---|
| - PPG-10 Butane diol | 0,588 |
| - polyglycéryl 3 diisostéarate | 0,511 |
| - Huile de ricin | 0,404 |

Par "compatibilisation", on entend une solubilisation totale ou partielle ou une dispersion homogène au microscope des fibres dans la phase grasse.

Les fibres peuvent être introduites dans la composition de l'invention suivant plusieurs procédés :
- Elles peuvent être solubilisées ou dispersées dans un ou plusieurs polyols selon l'invention, puis le mélange obtenu peut être ensuite solubilisé ou dispersé, en l'absence d'émulsionnant, dans la phase grasse de la composition, liquide ou rendue liquide par chauffage, si nécessaire (à condition de ne chauffer qu'à une température inférieure à celles de fusion ou de ramollissement des fibres) ;
- Le ou les polyols selon l'invention peuvent être, dans un premier temps, solubilisés ou dispersés dans la phase grasse de la composition, puis les fibres peuvent être solubilisées ou dispersées dans le mélange polyols/phase grasse.

De préférence, on utilise le premier mode d'incorporation des fibres, en utilisant un mélange 50/50 en poids de polyol et de fibres. Le mélange peut être réalisé à l'aide d'une turbine fournissant suffisamment d'énergie mécanique pour mouiller parfaitement les fibres comme la turbine Turbotest-Rayneri vendue par la société VMI (Montaigu, France).

Le ou les polyols compatibilisant les fibres avec la phase grasse peuvent être présents en une quantité allant de 0,1 à 95 % du poids total de la composition et mieux en une quantité allant de 1 à 50 %.

Cette composition peut être utilisée telle quelle ou bien être incorporée dans une composition plus complexe. Elle est notamment non collante au toucher, non grasse et douce à l'application, s'étalant bien, tout en étant d'un aspect homogène.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 20 mm, de préférence de 10 nm à 5 mm et mieux de 0,1 mm à 1,5 mm. Leur section peut être comprise dans un cercle de diamètre allant de 2 nm à 100µm, de préférence allant de 20 nm à 20 µm et mieux de 500nm à 20 µm. Le poids ou titre des fibres est souvent donné en denier ou décitex et représente le poids en gramme pour 9 km de fil. De préférence, les fibres selon l'invention ont un titre choisi dans la gamme allant de 0,15 à 30 deniers et mieux de 0,18 à 18 deniers.

Pour obtenir un maquillage brillant, ce qui tout particulièrement recherché pour le maquillage des ongles et des lèvres, on utilise avantageusement des fibres courtes ayant en particulier une longueur allant de 1 nm à 200µm. En revanche, pour un maquillage mat, ce qui surtout recherché pour le maquillage du visage (notamment pour une poudre ou un fond de teint), on utilise de préférence des fibres longues, ayant notamment une longueur supérieure à 200µm.

Les fibres peuvent être celles utilisées dans la fabrication des textiles et notamment des fibres de soie, de coton, de laine, de lin, des fibres de cellulose extraites notamment du bois, des légumes ou des algues, de polyamide (Nylon ®), de rayonne, de viscose, d'acétate notamment d'acétate de rayonne, de cellulose ou de soie, de poly-p-phénylène téréphtamide notamment de Kevlar ®, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon ®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et ε-caprolactone (Monocryl de chez Johnson & Johnson); les fibres synthétiques résorbables du type copolymère d'acide lactique et d'acide glycolique (Vicryl de chez Johnson & Johnson) ; les fibres de polyester téréphtalique (Ethibond de chez Johnson & Johnson) et les fils d'acier inoxydable (Acier de chez Johnson & Johnson) notamment pour une application en vernis à ongles.

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet anti-statique (par exemple le R-STAT de chez Rhodia) ou un autre polymère permettant une organisation particulière des fibres (traitement de surface spécifique) ou traitement de surface induisant des effets de couleurs/hologrammes (fibre Lurex de chez Sildorex, par exemple).

De préférence, on utilise des fibres d'origine synthétiques et en particulier des fibres organiques, comme celles utilisées en chirurgie.

Les fibres utilisables dans la composition selon l'invention sont préférentiellement des fibres de polyamide ou de poly-p-phénylène téréphtamide. Leur longueur (L) peut aller de 0,1 à 5 mm, de préférence de 0,25 à 1,6 mm et leur diamètre moyen (D) peut aller de 5 à 50 µm. En particulier, on peut utiliser les fibres de polyamide commercialisées par les Etablissements P. Bonte sous le nom Polyamide 0,9 Dtex 0,3 mm, ayant un diamètre moyen de 6µm, un poids d'environ (0,9 dtex) et une longueur allant de 0,3mm à 1,5mm. On peut aussi utiliser les fibres de poly-p-phénylène téréphtamide de diamètre moyen de 12 µm et de longueur d'environ 1,5 mm comme celles vendues sous le nom de Kevlar Floc par la société Du Pont Fibres.

La concentration en fibres est fonction de l'application spécifique et du type de produit envisagé. Pour un produit de maquillage du visage du type fond de teint ou des lèvres (du type rouge à lèvres) la concentration en fibres peut aller de 0,1 à 20% du poids total de la composition, de préférence de 0,5 à 10%. Pour un effet spécial notamment de maquillage du corps, des ongles ou des cheveux, la quantité de fibres peut aller jusqu'à 30% du poids total de la composition.

La composition de l'invention peut se présenter sous forme de pâte, de solide, de crème plus ou moins fluide voire même de lotion anhydre. Elle peut être un gel anhydre, rigide ou souple, éventuellement coulé en stick ou en coupelle. De préférence, elle se présente sous forme coulée.

La phase grasse de la composition de l'invention peut contenir un ou plusieurs corps gras liquides à température ambiante et pression atmosphérique, appelés huiles. Ces huiles peuvent être des huiles hydrocarbonées d'origine animale, végétale, minérale ou synthétique, des huiles siliconées et/ou fluorées et leurs mélanges.

Par "huile hydrocarbonée", on entend une huile contenant majoritairement des atomes de carbone et des atomes d'hydrogène et en particulier des chaînes alkyle ou alcényle comme les alcanes ou alcènes mais aussi une huile à chaîne alkyle ou alcényle comportant un ou des groupements éther, ester ou acide caboxylique.

Comme huiles utilisables dans la composition selon l'invention, on peut citer notamment:
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le parléam ;
- les esters et les éthers de synthèse notamment d'acides gras comme les huiles de formule R₁COOR₂ dans laquelle R₁ représente le reste d'un acide gras supérieur comportant de 7 à 29 atomes de carbone et R₂ représente une chaîne hydrocarbonée contenant de 3 à 30 atomes de carbone comme par exemple l'huile de Purcellin, l'isononanoate d'isononyle, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol comme le tétraiso stéarate de pentaérythrytyle ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényltriméthylsiloxy diphényl-siloxanes, les diphényl diméthicones, les diphénylméthyldiphényl trisiloxanes, les 2-phényléthyl triméthyl-siloxysilicates,
- leurs mélanges.

Ces huiles peuvent représenter de 0,2 à 99,85 % du poids total de la composition, de préférence de 1 à 80 % et mieux de 10 à 80 %.

La composition de l'invention peut comprendre, en outre, tout ingrédient usuellement utilisé dans le domaine concerné, tel que des matières colorantes comme les pigments, les nacres, les colorants solubles dans la phase grasse liquide ou dans les polyols selon l'invention, des antioxydants, des huiles essentielles, des conservateurs, des parfums, des polymères liposolubles notamment hydrocarbonés tels que les polyalkylènes ou le polylaurate de vinyle, des gélifiants de phase grasse liquide, des agents structurants de la phase grasse liquide comme les cires, les gommes et les charges, des agents dispersants de pigments, des actifs cosmétiques ou dermatologiques comme par exemple des émollients, des hydratants (eau), des vitamines, de la lanoline liquide, des acides gras essentiels, des filtres solaires lipophiles ou hydrophiles et leurs mélanges. Ces ingrédients peuvent être présents dans la composition au taux usuellement utilisés dans les domaines considérés.

Bien entendu l'homme du métier veillera à choisir les éventuels ingrédients complémentaires et/ou leur quantité de telle manière que les propriétés avantageuses de la composition selon l'invention ne soient pas ou substantiellement pas, altérées par l'adjonction envisagée.

Avantageusement la composition contient des cires en vue de la rigidifier. Les cires peuvent être hydrocarbonées, fluorées et/ou siliconées et être d'origine végétale, minérale, animale et/ou synthétique. En particulier, les cires présentent une température de fusion supérieure à 45 °C.

Comme cire utilisable dans l'invention, on peut citer des cires hydrocarbonées (ne contenant que des atomes de carbone et d'hydrogène), siliconées et/ou fluorées, comportant éventuellement des fonctions ester, hydroxyle ou thiol. A titre d'exemple, on peut citer, la lanoline, la cire d'abeilles, la cire de Carnauba ou de Candellila, la paraffine, les cires de lignite ou microcristalline, la cérésine ou l'ozokérite ; les cires synthétiques comme les cires de polyéthylène, les cires de silicones comme les alkyl ou alkoxydiméticone ayant de 16 à 45 atomes de carbone, les cires de Fischer-Tropsch et leurs mélanges.

La nature et la quantité des cires sont fonction des propriétés mécaniques et de textures recherchées. A titre indicatif, la composition peut contenir de 0 à 50 % en poids de cires, par rapport au poids total de la composition et mieux de 5 à 30 %. Ces cires sont, en outre, des agents structurants de la composition.

La composition de l'invention peut comprendre, avantageusement une phase particulaire, généralement présente à raison de 0 à 40 % du poids total de la composition, de préférence de 0,5 à 25 %, et mieux de 1 à 25 % et qui peut comprendre des pigments et/ou des nacres et/ou des charges habituellement utilisés dans les compositions cosmétiques ou dermatologiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition et font notamment partie des agents structurants susceptibles de conduire à une forme solide.

Les pigments peuvent être présents dans la composition à raison de 0,05 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium (DC Red N°21 ou FDC Yellow N° 6).

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0,5 à 15 %. On peut notamment citer le talc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le Polytrap (Dow Corning) et les microbilles de résine de silicone (Tospearl de Toshiba, par exemple).

La composition selon l'invention peut se présenter sous la forme d'une composition dermatologique ou de soin de la peau ou sous forme d'une composition de protection solaire ou de démaquillage. Elle se présente alors sous forme non colorée, contenant éventuellement des actifs cosmétiques ou dermatologiques. Elles peuvent alors être utilisées comme base de soin pour la peau ou les lèvres (baumes à lèvres, protégeant les lèvres du froid et/ou du soleil et/ou du vent), crème de soin de jour ou de nuit.

La composition de l'invention peut également se présenter sous la forme d'un produit coloré de maquillage de la peau, en particulier un fond de teint, un blush, un fard à joues ou à paupières ou de maquillage des lèvres comme un rouge à lèvres ou un brillant à lèvres, présentant éventuellement des propriétés de soin ou de traitement ou encore sous forme d'eye liner, de mascara, ou de vernis à ongles.

L'invention peut, en outre, se présenter sous forme de shampooing traitant ou non, colorant ou non, de produit après shampooing.

Bien entendu la composition de l'invention doit être cosmétiquement ou dermatologiquement acceptable, à savoir non toxique et susceptible d'être appliquée sur la peau ou les lèvres ainsi que sur les phanères d'êtres humains.

La composition selon l'invention peut être fabriquée par les procédés connus, généralement utilisés dans le domaine cosmétique ou dermatologique.

L'invention a encore pour objet un procédé cosmétique de soin ou de traitement des matières kératiniques et notamment de la peau ou des lèvres des êtres humains, comprenant l'application sur matières kératiniques de la composition en particulier cosmétique telle que définie ci-dessus.

L'invention a encore pour objet l'utilisation cosmétique de fibres dans une composition anhydre de soin ou de maquillage des matières kératiniques, contenant une phase grasse et au moins un polyol liquide à température ambiante, pour conférer à ladite composition de la tenue dans le temps notamment à la pression et au frottement et/ou de la brillance.

L'invention a encore pour objet un procédé cosmétique pour conférer de la tenue dans le temps et/ou de la brillance à une composition anhydre de soin ou de maquillage, contenant une phase grasse et au moins un polyol liquide à température ambiante, consistant à introduire dans ladite composition des fibres.

L'invention est illustrée plus en détail dans les exemples suivants. Les pourcentages sont à température ambiante donnés en poids.

### Exemple 1 : Rouge à lèvres renfermant des fibres de polyamide

. Cire de polyéthylène 7 %
. Cire microcristalline 7 %
. Huile de sésame 24 %
. Huile de jojoba 24 %
. Phényltriméthicone 3 %
. Fibre de polyamide (0.3mm de long) 5 %
. Pigments 9,5 %
. Lanoline 10,5 %
. Glycérine 10 %

*Préparation* : On introduit à température ambiante (25°C) les fibres dans la glycérine et on soumet l'ensemble à une agitation de 1000 tr/min pendant 30 min avec une turbine Rayneri. Par ailleurs, on chauffe le mélange de cires et d'huile à une température de 100°C, puis on introduit les pigments dans le mélange fondu, sous agitation dans une turbine tricylincre. On introduit le mélange glycérine/fibres dans le mélange cire/huile/pigments. Le tout est mélangé dans un poelon (à 100°C) sous agitation magnétique pendant 20min. Le mélange est ensuite coulé dans un moule pré-chauffé à 45°C, pour donner un stick.

On obtient un rouge à lèvres brillant ayant de bonne propriété de tenue et de résistance au frottement et à la pression, assurant un maquillage homogène et esthétique. En particulier, les propriétés de sans transfert de ce rouge à lèvres ont été comparées à celles d'un rouge à lèvres selon l'art antérieur contenant les mêmes ingrédients à l'exception des fibres, la teneur en fibres étant compensée par la teneur glycérine. L'application de ces 2 compositions est effectuée en comparatif par demi-lèvre. Après un séchage à l'air ambiant de 2 min, les testrices effectuent un « bisous » sur une feuille de papier d'imprimerie. Elles ré-appliquent du rouge à lèvres par demi-lèvres puis effectuent un « bisous » sur la feuille de papier après un séchage à l'air ambiant de 10 min. Elles effectuent un autre « bisous » sur la feuille de papier après un séchage à l'air ambiant de 50 min. L'appréciation du sans transfert est visuelle.

100 % des testrices ont noté des propriétés de sans transfert très supérieures pour le rouge à lèvres selon l'invention par rapport à celui de l'art antérieur, pour les 3 tests de « bisous ».

### Exemple 2 : Fond de teint anhydre renfermant des fibres de polyamide

. Cire microcristalline 3 %
. Cire de Carnauba 5.5 %
. Octyl palmitate 27.5 %
. Octyl dodécanol 10 %
. Fibres de polyamide (0.3mm de long) 3 %
. Glycérine 7 %
. Pigmentes 10 %
. TiO2 nanométrique 9 %
. Silicate de Magnésium 8 %
. Poudre de Nylon 9,5 %
. Poudre de silice 4 %
. Actifs 2,5 %

Ce fond de teint est réalisé comme le rouge à lèvres de l'exemple 1, avec introduction des actifs en dernier puis coulage dans un boîtier approprié. Le fond de teint obtenu confère un maquillage homogène esthétique, doux, non gras, ne transférant pas même sous la pression.

### Exemple 3 : Rouge à lèvres renfermant des fibres de Kevlar Floc

. Cire d'abeilles oxypropylénée 14,5 %
. Cire microcristalline 3 %
. Cire de lanoline oxypropylénée 2 %
. Huile de sésame 18 %
. Huile d'arara 10 %
. Erucate d'oléyle 10 %
. Lanoline 20 %
. Lanoline acétylée 6 %
. Fibres de Kevlar (1,5 mm de long) 2 %
. Propylène glycol 7 %
. Pigments 10,5 %

Ce rouge à lèvres en stick est réalisé comme le rouge à lèvres de l'exemple 1. Le rouge à lèvres obtenu confère un maquillage homogène esthétique, doux, non gras, ne transférant pas même sous la pression.

### Exemple 4 : Rouge à lèvres renfermant des fibres de polyamide

. Cire d'abeilles polyglycérolée (3 moles) 4,4 %
. Cire microcristalline 11 %
. Cire de lanoline oxypropylénée 6,2 %
. Huile de sésame 12,4 %
. Huile d'arara 12,4 %
. Erucate d'oléyle 12,4 %
. Lanoline 20 %
. Lanoline acétylée 6,2 %
. Fibres de polyamide (0,9 Dtex 0,3) 5 %
. Pigments qsp 100 %

Ce stick de rouge à lèvres a été comparé à un stick de rouge à lèvres de même composition dans laquelle les 5 % de fibres ont été remplacées par 5 % de poudre sphérique de copolymère de méthacrylate de lauryl/méthacrylate d'éthylène glycol (Polytrap 6603 vendu par APS) et a été jugé moins brillant que le rouge à lèvres de l'invention par un panel d'experts. La poudre Polytrap a un diamètre D de 0,1 à 1 µm et un facteur de forme L/D de 0.

## Revendications

1. Composition anhydre de soin ou de maquillage, contenant des fibres compatibilisées avec une phase grasse par au moins un polyol liquide à température ambiante, les fibres ayant une longueur allant de 10 nm à 5 mm, et une section comprise dans un cercle de diamètre allant de 2 nm à 100µm.

2. Composition anhydre de soin ou de maquillage, contenant des fibres compatibilisées avec une phase grasse par au moins un polyol liquide à température ambiante, les fibres ayant une longueur L et un diamètre D tel que le rapport L/D est choisi dans la gamme allant de 3,5 à 2 500.

3. Composition selon la revendication 1 ou 2, **caractérisée en ce que** le polyol présente une valeur d'IOB allant de 1 à 7 et de préférence de 1,5 à 5,5.

4. Composition selon l'une des revendications précédentes, **caractérisée en ce que** le polyol est choisi parmi le propylène glycol, le butylène glycol, l'isoprène glycol, le pentylène glycol, l'hexylène glycol, les polyéthylène glycols ayant de 4 à 8 motifs éthylène glycol, le glycérol, le panthénol, et leurs mélanges.

5. Composition selon l'une des revendications précédentes, **caractérisée en ce que** le polyol est présent en une quantité allant de 0,1 à 95 % du poids total de la composition et mieux en une quantité allant de 1 à 50 %.

6. Composition selon l'une des revendications précédentes, **caractérisée en ce que** les fibres sont choisies parmi les fibres de soie, de coton, de laine, de lin, des fibres de cellulose extraites notamment du bois, des légumes ou des algues, de polyamide, de rayonne, de viscose, d'acétate notamment d'acétate de rayonne, de cellulose ou de soie, de poly-p-phénylène téréphtamide, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon ®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, des fibres chirurgicales.

7. Composition selon l'une quelconque des revendications précédentes, **caractérisée en ce que** les fibres sont des fibres d'origine synthétique.

8. Composition selon l'une quelconque des revendications précédentes, **caractérisée en ce que** les fibres sont des fibres de polyamide ou de poly-p-phénylène téréphtamide.

9. Composition selon l'une quelconque des revendications précédentes, **caractérisée en ce que** les fibres ont une longueur allant de 0,25 à 1,6 mm.

10. Composition selon l'une des revendications précédentes, **caractérisée en ce que** la phase grasse contient au moins une huile liquide à température ambiante choisie parmi les huiles hydrocarbonées d'origine animale, végétale ou minérale, les huiles siliconées et/ou fluorées et leurs mélanges.

11. Composition selon l'une des revendications précédentes, **caractérisée en ce qu'**elle contient au moins une huile choisie parmi le perhydrosqualène ; les triglycérides des acides heptanoïque ou octanoïque ou encore les huiles de tournesol, de maïs, de soja, de courge, de pépins de raisin, de sésame, de noisette, d'abricot, de macadamia, d'arara, de ricin, d'avocat, les triglycérides des acides caprylique/caprique, l'huile de jojoba, de beurre de karité ; les huiles de paraffine et leurs dérivés, la vaseline, les polydécènes, le polyisobutène hydrogéné tel que le parléam ; l'huile de Purcellin, le myristate d'isopropyle, l'isononanoate d'isononyle, le palmitate d'éthyl-2hexyle, le stéarate d'octyl-2-dodécyle, l'érucate d'octyl-2-dodécyle, l'isostéarate d'isostéaryle ; l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; le dioctanoate de propylène glycol, le diheptanoate de néopentylglycol, le diisononanoate de diéthylèneglycol ; les esters du pentaérythritol ; l'octyldodécanol, le 2-butyloctanol, le 2-hexyldécanol, le 2-undécylpentadécanol, l'alcool oléique ; les polydimethylsiloxanes (PDMS) volatiles ou non, linéaires ou cycliques, liquides à température ambiante ; les phényl triméthicones, les phényltriméthylsiloxy-diphénylsiloxanes, les diphényl diméthicones, les diphényl-méthyldiphényl trisiloxanes, les 2-phényl-éthyl triméthylsiloxysilicates, et leurs mélanges.

12. Composition selon l'une des revendications précédentes, **caractérisée en ce qu'**elle contient, en outre, au moins une charge particulaire.

13. Composition selon l'une des revendications précédentes, **caractérisée en ce qu'**elle se présente sous forme de un gel anhydre, rigide ou souple, éventuellement coulé en stick ou en coupelle.

14. Composition selon l'une des revendications précédentes, **caractérisée en ce qu'**elle contient, en outre, au moins un ingrédient choisi parmi les matières colorantes, les antioxydants, les huiles essentielles, les conservateurs, les parfums, les polymères liposolubles, les gélifiants de phase grasse liquide, les cires, les gommes, les charges, les dispersants, les actifs cosmétiques ou dermatologiques et leurs mélanges.

15. Composition selon l'une des revendications précédentes, **caractérisée en ce qu'**elle constitue un rouge à lèvres ou un brillant à lèvres, un fond de teint, un mascara, un eye liner, un fard à joues ou à paupières, un produit de maquillage du corps, un produit de soin ou de protection solaire.

16. Procédé cosmétique de soin ou de traitement des matières kératiniques des êtres humains, comprenant l'application sur ces matières kératiniques de la composition cosmétique selon l'une quelconque des revendications précédentes.

17. Utilisation cosmétique de fibres dans une composition de soin ou de maquillage des matières kératiniques, contenant au moins un polyol liquide à température ambiante et une phase grasse, pour lui conférer de la tenue dans le temps notamment à la pression et au frottement et/ou de la brillance, ces fibres ayant une longueur allant de 10 nm à 5 mm et un diamètre moyen allant de 2nm à 100 µm.

18. Utilisation cosmétique de fibres dans une composition de soin ou de maquillage des matières kératiniques, contenant au moins un polyol liquide à température ambiante et une phase grasse, pour lui conférer de la tenue dans le temps notamment à la pression et au frottement et/ou de la brillance, ces fibres ayant une longueur L et un diamètre D tel que le rapport UD est choisi dans la gamme allant de 3,5 à 2 500.

## Patentansprüche

1. Wasserfreie Zusammensetzung zur Pflege oder zum Schminken, die Fasern enthält, die mit mindestens einem bei Raumtemperatur flüssigen Polyol mit einer flüssigen Fettphase kompatibel gemacht wurden, wobei die Fasern eine Länge von 10 nm bis 5mm besitzen und einen Querschnitt aufweisen, der in einen Kreis von 2 nm bis 100 µm einbeschrieben werden kann.

2. Wasserfreie Zusammensetzung zur Pflege oder zum Schminken, die Fasern enthält, die mit mindestens einem bei Raumtemperatur flüssigen Polyol mit einer flüssigen Fettphase kompatibel gemacht wurden, wobei die Fasern eine solche Länge und einen solchen Durchmesser D aufweisen, dass das Verhältnis L/D im Bereich von 3,5 bis 2 500 liegt.

3. Zusammensetzung nach Anspruch 1 oder 2, **dadurch gekennzeichnet, dass** das Polyol eine IOB-Wert von 1 bis 7 und vorzugsweise 1,5 bis 5,5 aufweist.

4. Zusammensetzung nach einem der vorhergehenden Ansprüche, **dadurch gekennzeichnet, dass** das Polyol unter Propylenglykol, Butylenglykol, Isoprenglykol, Pentylenglykol, Hexylenglykol, Polyethylenglykolen mit 4 bis 8 Ethylenglykoleinheiten, Glycerin, Panthenol und deren Gemischen ausgewählt ist.

5. Zusammensetzung nach einem der vorhergehenden Ansprüche, **dadurch gekennzeichnet, dass** das Polyol in einer Menge von 0,1 bis 95 % des Gesamtgewichts der Zusammensetzung und besser in einer Menge von 1 bis 50 % enthalten ist.

6. Zusammensetzung nach einem der vorhergehenden Ansprüche, **dadurch gekennzeichnet, dass** die Fasern unter den Seidenfasern, Baumwollfasern, Wollfasern, Leinenfasern, Cellulosefasern, die insbesondere aus Holz, Gemüse oder Algen gewonnen wurden, Polyamidfasern, Reyonfasern, Viskosefasern, Acetatfasern und insbesondere Fasern aus Reyonacetat, Celluloseacetat oder Seidenacetat, Fasern aus Poly(p-phenylen-terephthalamid), Acrylfasern, insbesondere Fasern aus Polymethylmethacrylat oder Poly-2-hydroxyethylmethacrylat, Polyolefinfasern und insbesondere Fasern aus Polyethylen oder Polypropylen, Glasfasern, Siliciumdioxidfasern, Aramidfasern, Kohlenstofffasern und insbesondere aus Kohlenstoff in Form von Graphit, Fasern aus Teflon®, Fasern aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern, Fasern aus Polymergemischen und chirurgischen Fasern ausgewählt sind.

7. Zusammensetzung nach einem der vorhergehenden Ansprüche, **dadurch gekennzeichnet, dass** die Fasern synthetischer Herkunft sind.

8. Zusammensetzung nach einem der vorhergehenden Ansprüche, **dadurch gekennzeichnet, dass** die Fasern Polyamidfasern oder Fasern aus Poly(p-phenylen-terephthalamid) sind.

9. Zusammensetzung nach einem der vorhergehenden Ansprüche, **dadurch gekennzeichnet, dass** die Fasern eine Länge von 0,25 bis 1,6 mm aufweisen.

10. Zusammensetzung nach dem vorhergehenden Anspruch, **dadurch gekennzeichnet, dass** die Fettphase mindestens ein bei Raumtemperatur flüssiges Öl enthält, das unter den Kohlenwasserstoffölen tierischer, pflanzlicher oder mineralischer Herkunft, Siliconölen und/oder fluorierten Ölen und deren Gemischen ausgewählt ist.

11. Zusammensetzung nach einem der vorhergehenden Ansprüche, **dadurch gekennzeichnet, dass** sie mindestens ein Öl enthält, das ausgewählt ist unter: Perhydrosqualen; Triglyceriden von Heptansäure oder Octansäure, Sonnenblumenöl, Maisöl, Sojaöl, Kürbiskernöl, Traubenkernöl, Sesamöl, Haselnussöl, Aprikosenkernöl, Macadamiaöl, Araraöl, Ricinusöl, Avocadoöl, Triglyceriden von Capryl/Caprinsäure, Jojobaöl, Sheabutter; Paraffinölen und deren Derivaten, Vaseline, Polydecenen und hydriertem Polyisobuten, beispielsweise Parleam; Purcellinöl, Isopropylmyristat, Isononylisononanoat, 2-Ethylhexylpalmitat, 2-Octyldodecylstearat, 2-Octyldodecylerucat, Isostearylisostearat; Isostearyllactat, Octylhydroxystearat, Octyldodecylhydroxystearat, Diisostearylmalat, Triisocetylcitrat, Heptanoaten, Octanoaten oder Decanoaten von Fettalkoholen; Propylenglykoldioctanoat, Neopentylglykoldiheptanoat, Diethylenglykoldiisononanoat; Estern von Pentaerythrit; Octyldodecanol, 2-Butyloctanol, 2-Hexyldecanol, 2-Undecylpentadecanol oder Oleylalkohol; geradkettigen oder cyclischen, bei Raumtemperatur flüssigen, flüchtigen oder nicht flüchtigen Polymethylsiloxanen (PDMS); Phenyltrimethiconen, Phenyltrimethylsiloxydiphenylsiloxanen, Diphenyldimethiconen, Diphenylmethyldiphenyltrisiloxanen, 2-Phenylethyltrimethylsiloxysilicaten und deren Gemischen.

12. Zusammensetzung nach einem der vorhergehenden Ansprüche, **dadurch gekennzeichnet, dass** sie ferner mindestens einen Füllstoff in Partikelform enthält.

13. Zusammensetzung nach einem der vorhergehenden Ansprüche, **dadurch gekennzeichnet, dass** sie in Form eines wasserfreien, starren oder weichen Gels vorliegt, das gegebenenfalls in Stiftform vorliegt oder in ein Tiegelchen gegossen wurde.

14. Zusammensetzung nach einem der vorhergehenden Ansprüche, **dadurch gekennzeichnet, dass** sie außerdem mindestens einen Bestandteil enthält, der unter den Farbmitteln, Antioxidantien, etherischen Ölen, Konservierungsmitteln, Parfums, fettlöslichen Polymeren, Gelbildnem für die flüssige Fettphase, Wachsen, Gummen/ Gummis, Füllstoffen, Dispergiermitteln, kosmetischen und dermatologischen Wirkstoffen und deren Gemischen ausgewählt ist.

15. Zusammensetzung nach einem der vorhergehenden Ansprüche, **dadurch gekennzeichnet, dass** sie als Lippenstift, Lipgloss, Make-up, Mascara, Eyeliner, Lidschatten, Wangenrouge, Produkt zum Schminken des Körpers, pflegendes Produkt oder Produkt zum Sonnenschutz vorliegt.

16. Kosmetisches Verfahren zur Pflege oder zur Behandlung von menschlichen Keratinsubstanzen, das urnfasst, eine kosmetische Zusammensetzung nach einem der vorhergehenden Ansprüche auf die Keratinsubstanzen aufzutragen.

17. Kosmetische Verwendung von Fasern in einer kosmetischen Zusammensetzung zur Pflege oder zum Schminken von Keratinsubstanzen, die mindestens ein bei Raumtemperatur flüssiges Polyol und eine Fettphase enthält, um ihr insbesondere gegenüber Druck und Reibung Langzeitbeständigkeit zu geben und/oder um ihr Glanz zu verleihen, wobei die Fasern eine Länge von 10 nm bis 5mm und einen mittleren Durchmesser von 2 nm bis 100 µm besitzen.

18. Kosmetische Verwendung von Fasern in einer kosmetischen Zusammensetzung zur Pflege oder zum Schminken von Keratinsubstanzen, die mindestens ein bei Raumtemperatur flüssiges Polyol und eine Fettphase enthält, um ihr insbesondere gegenüber Druck und Reibung Langzeitbeständigkeit zu geben und/oder um ihr Glanz zu verleihen, wobei die Fasern eine solche Länge L und einen solchen Durchmesser D aufweisen, dass das Verhältnis L/D im Bereich von 3,5 bis 2500 liegt.

## Claims

1. Anhydrous care or make-up composition containing fibres which are compatibilized with a fatty phase by means of at least one polyol that is liquid at room temperature, the fibres having a length ranging from 10 nm to 5 mm, and a cross section included in a circle having a diameter ranging from 2 nm to 100 µm.

2. Anhydrous care or make-up composition containing fibres which are compatibilized with a fatty phase by means of at least one polyol that is liquid at room temperature, the fibres having a length L and a diameter D such that the ratio L/D is chosen in the range extending from 3.5 to 2500.

3. Composition according to Claim 1 or 2, **characterized in that** the polyol has an IOB value ranging from 1 to 7 and preferably from 1.5 to 5.5.

4. Composition according to one of the preceding claims, **characterized in that** the polyol is chosen from propylene glycol, butylene glycol, isoprene glycol, pentylene glycol, hexylene glycol, polyethylene glycols containing from 4 to 8 ethylene glycol units, glycerol and panthenol, and mixtures thereof.

5. Composition according to one of the preceding claims, **characterized in that** the polyol is present in an amount ranging from 0.1 to 95% relative to the total weight of the composition, and better still in an amount ranging from 1 to 50%.

6. Composition according to one of the preceding claims, **characterized in that** the fibres are chosen from silk fibre, cotton fibre, wool fibre, flax fibre, cellulose fibres extracted in particular from wood, from plants or from algae, polyamide fibre, rayon fibre, viscose fibre, acetate fibre, in particular rayon acetate fibre, cellulose acetate fibre or silk acetate fibre, poly-p-phenylene terephthamide fibre, acrylic fibre, in particular polymethyl methacrylate fibre or poly(2-hydroxyethyl methacrylate) fibre, polyolefin fibre, and in particular polyethylene or polypropylene fibre, glass fibre, silica fibre, aramid fibre, carbon fibre, in,particular in graphite form, Teflon® fibre, insoluble collagen fibre, polyester fibre, polyvinyl chloride or polyvinylidene chloride fibre, polyvinyl alcohol fibre, polyacrylonitrile fibre, chitosan fibre, polyurethane fibre, polyethylene phthalate fibre, or fibres formed from a mixture of polymers, and surgical fibres.

7. Composition according to any one of the preceding claims, **characterized in that** the fibres are fibres of synthetic origin.

8. Composition according to any one of the preceding claims, **characterized in that** the fibres are polyamide fibres or poly-p-phenylene terephthamide fibres.

9. Composition according to any one of the preceding claims, **characterized in that** the fibres have a length ranging from 0.25 to 1.6 mm.

10. Composition according to any one of the preceding claims, **characterized in that** the fatty phase contains at least one oil that is liquid at room temperature, chosen from hydrocarbon-based oils of animal, plant or mineral origin, silicone oils and/or fluoro oils, and mixtures thereof.

11. Composition according to one of the preceding claims, **characterized in that** it contains at least one oil chosen from perhydrosqualene; heptanoic or octanoic acid triglycerides or alternatively sunflower oil, corn oil, soybean oil, marrow oil, grapeseed oil, sesame oil, hazelnut oil, apricot oil, macadamia oil, arara oil, castor oil, avocado oil, caprylic/capric acid triglycerides, jojoba oil or karite butter; liquid paraffin and derivatives thereof, petroleum jelly, polydecenes or hydrogenated polyisobutene such as parleam; purcellin oil, isopropyl myristate, isononyl isononanoate, 2-ethylhexyl palmitate, 2-octyldodecyl stearate, 2-octyldodecyl erucate, isostearyl isostearate; isostearyl lactate, octyl hydroxystearate, octyldodecyl hydroxystearate, diisostearyl malate, triisocetyl citrate, fatty alkyl heptanoates, octanoates and decanoates; propylene glycol dioctanoate, neopentyl glycol diheptanoate, diethylene glycol diisononanoate; pentaerythritol esters; octyldodecanol, 2-butyloctanol, 2-hexyldecanol, 2-undecylpentadecanol or oleyl alcohol; volatile or non-volatile, linear or cyclic polydimethylsiloxanes (PDMSs) which are liquid at room temperature; phenyl trimethicones, phenyltrimethylsiloxydiphenylsiloxanes, diphenyl dimethicones, diphenylmethyldiphenyltrisiloxanes, 2-phenylethyl trimethylsiloxysilicates, and mixtures thereof.

12. Composition according to one of the preceding claims, **characterized in that** it also contains at least one particulate filler.

13. Composition according to one of the preceding claims, **characterized in that** it is in the form of a rigid or supple anhydrous gel, optionally cast as a stick or dish.

14. Composition according to one of the preceding claims, **characterized in that** it also contains at least one ingredient chosen from dyestuffs, antioxidants, essential oils, preserving agents, fragrances, liposoluble polymers, agents for gelling the liquid fatty phase, waxes, gums, fillers, dispersants, cosmetic or dermatological active agents and mixtures thereof.

15. Composition according to one of the preceding claims, **characterized in that** it constitutes a lipstick or a lip gloss, a foundation, a mascara, an eyeliner, a face powder, an eyeshadow, a make-up product for the body or an antisun care product.

16. Cosmetic care or treatment process for the keratin substances of human beings, comprising the application to these keratin substances of the cosmetic composition according to any one of the preceding claims.

17. Cosmetic use of fibres in a care or make-up composition for keratin substances, containing at least one polyol that is liquid at room temperature and a fatty phase, to give it staying power over time, in particular at pressure and while rubbing, and/or gloss, these fibres having a length ranging from 10 nm to 5 mm and an average diameter ranging from 2 nm to 100 µm.

18. Cosmetic use of fibres in a care or make-up composition for keratin substances, containing at least one polyol that is liquid at room temperature and a fatty phase, to give it staying power over time, in particular at pressure and while rubbing, and/or gloss, these fibres having a length L and a diameter D such that the ratio L/D is chosen in the range extending from 3.5 to 2500.
